# EUROPÄISCHE PATENTANMELDUNG

(11) **EP 3 417 888 A1**
(43) Veröffentlichungstag der Anmeldung: **26.12.2018**
(21) Anmeldenummer: 17177756.8
(22) Anmeldetag: 25.06.2017
(51) Int. Cl.: A61L 27/38, C12N 5/077, G01N 33/68

(54) **VERFAHREN ZUM HERSTELLEN VON TRANSPLANTIERBAREM KNORPELGEWEBE**

(71) Anmelder: co.don AG, 14513 Teltow (DE)
(72) Erfinder: ROEL, Giadiata, 10439 Berlin (DE); KAPS, Christian, 13086 Berlin (DE); ESCHEN, Claudia, 10439 Berlin (DE)
(74) Vertreter: Simandi, Claus

(57) **Zusammenfassung**

Die vorliegende Erfindung betrifft ein Verfahren zum Herstellen von Knorpelgewebe mittels Selektion und Auswahl aus Chrondozyten oder Sphäroiden und dessen Verwendung als pharmazeutische Zusammensetzung, Arzneimittel, Transplantat. Insbesondere betrifft die Erfindung Marker zum Identifizieren geeigneter Chondrozyten zum Herstellen von Knorpelgewebe, insbesondere zu Zwecken der Transplantation.

## Beschreibung

Die vorliegende Erfindung betrifft ein Verfahren zum Herstellen von Knorpelgewebe mittels Selektion und Auswahl aus Chondrozyten oder Sphäroiden und dessen Verwendung als pharmazeutische Zusammensetzung, Arzneimittel oder Transplantat. Insbesondere betrifft die Erfindung Marker zum Identifizieren geeigneter Chondrozyten zum Herstellen von Knorpelgewebe, insbesondere zu Zwecken der Transplantation.

Obwohl Gelenkknorpel eine bemerkenswerte Widerstandsfähigkeit zeigt, hat dieses Gewebe kein oder ein sehr geringes Vermögen zur Selbstreparatur und Regeneration als auch unbehandelte Läsionen können beispielsweise Osteoarthritis zur Folge haben. Dieses geringe Potential zur spontanen Regeneration führte zur Entwicklung von Zelltherapien, wie der autologen Chondrozytentransplantation (ACT), im Bestreben, eine funktionelle und schmerzlose Reparatur von Gelenkknorpeldefekten bereit zu stellen. Es besteht ein hoher Bedarf an Verfahren zur Knorpelregeneration, beispielsweise bei jungen aktiven Patienten mit traumatischen Läsionen oder sogar mit Symptomen einer Knorpeldegeneration oder zur Behandlung von Knorpeldefekten.

Biochemische und molekulare Studien mit humanen Chondrozyten wurden von einer Reihe von Faktoren behindert, wie der mangelnden Verfügbarkeit von Humangewebe in Verbindung mit der sehr geringen Anzahl von Zellen, die in einer Biopsie verfügbar sind, der begrenzten Proliferationskapazität und der hohen phänotypischen Instabilität kultivierter Chondrozyten.

Ein Chondrozyt (Knorpelzelle), ist eine aus Chondroblasten hervorgehende und im Knorpelgewebe ansässige Zelle. Zusammen mit den Interzellularsubstanzen (extrazelluläre Matrix (EZM)), bilden die Chondrozyten die Hauptbestandteile des Knorpels, insbesondere Gelenkknorpel.

Es ist bereits bekannt, durch Mimikry bestimmter Prozesse der Embryonalentwicklung, z.B. menschliche Knorpelzellen dreidimensional auf einer Agaroseunterlage zu kultivieren, so dass Zellaggregate entstehen, die in ihrem Differenzierungsstatus den Monolayerzellen überlegen sind und z.B. knorpelähnliche Eigenschaften aufweisen. Diese Eigenschaften, die möglichst genau das native Gelenkknorpelgewebe widerspiegeln, sind gekennzeichnet durch die Expression von Kollagen II (Hauptstrukturprotein der extrazellulären Matrix des hyalinen Knorpels), von Proteoglykanen, z.B. Aggrecan und dem intrazellulären chondrozytenspezifischen Protein S100.

Darüber hinaus ist es wünschenswert, dass die Expression von Kollagen I, welches während der Zellvermehrungsphase in der Monolayerkultur zwangsläufig hochreguliert wird, in den Zellaggregaten wieder reduziert wird, da dieses Protein im nativen Gelenkknorpel nicht vorkommt. Die so generierten Zellaggregate (Sphäroide) werden seit 2002 von der Anmelderin zur Behandlung und Therapie von verletzungsbedingten Knorpeldefekten bei jüngeren Patienten verwendet. Dazu wird dem Patienten körpereigenes Gelenkknorpelgewebe und zwar eine Biopsie hyalinen Knorpels aus einem gesunden Knorpelareal entnommen. Durch enzymatischen Verdau des Knorpelgewebes mit einer Kollagenaselösung werden die im Knorpelgewebe befindlichen Knorpelzellen (Chondrozyten) isoliert und mittels Standardzellkulturbedingungen in Zellkulturflaschen (Monolayerkultur) in der Gegenwart von autologem Serum vermehrt, und ausreichend Chondrozyten zur Herstellung von transplantierbarem Knorpelgewebe zur Verfügung gestellt. Die vermehrten Zellen bilden Zellaggregate, so genannte Sphäroiden, die transplantiert werden können. Jedoch sind die derart hergestellten Zellaggregate in ihrem Differenzierungsstatus begrenzt und zeigen zumeist eine relativ schwache lokale Expression des wichtigen Kollagens II und eine marginale Synthese von Proteoglykanen bei relativ starker Expression des unerwünschten Kollagen I. Um die Differenzierung dieser Zellaggregate weiter zu verbessern, besteht die Möglichkeit das Kulturmedium mit bestimmten bioaktiven Substanzen anzureichern. Dazu zählen vorranging die vielfach in der Literatur beschriebenen Wachstumsfaktoren TGF-β1 - 3 sowie L-Ascorbinsäure (Vitamin C) als Kofaktor für die Kollagensynthese. Allerdings reicht diese biochemische Stimulation zum einen oftmals nicht aus, um die Differenzierung der Knorpelzellen im 3D Zellaggregat soweit zu induzieren, dass knorpeltypische Konstrukte entstehen und zum anderen ist der Einsatz von Wachstumsfaktoren wie TGF-β insbesondere für eine klinische Anwendung am Menschen fragwürdig.

Die Kultivierung von humanen Zellen in Form von dreidimensionalen Zellaggregaten, z.B. in Form von Sphäroiden, ist bereits zur klinischen Anwendung am Menschen, z.B. als autologes Knorpeltransplantat in DE 100 13 223 beschrieben und betrifft ein Verfahren zur in vitro Herstellung von dreidimensionalem Knorpel- und Knochengewebe aus Knochen-, Knorpel- oder mesenchymalen Stammzellen. Dabei werden die Zellen zunächst in einer Monolayerkultur und anschließend in Suspension so lange kultiviert, bis ein Zellaggregat entsteht, das zumindest 40 Vol % extrazelluläre Matrix beinhaltet, in welches differenzierte Zellen eingebettet sind.

In dem Verfahren werden Zellaggregate dadurch hergestellt, dass Zellen für mindestens 1-2 Wochen in Agarose beschichteten Zellkulturgefäßen kultiviert werden.

US 7,887,843 B2 offenbart ein Verfahren zur in vitro Erzeugung von dreidimensionalem Knorpel- und Knochengewebe, wobei transplantierbare Sphäroide aus Knochen-, Knorpel- oder mesenchymalen Stammzellen dadurch hergestellt werden, dass 1 x 10⁵ Zellen für mindestens zwei Wochen kultiviert werden.

Anderer et al. (Journal of Bone and Mineral Research (2002), New York, NY, US, Bd. 17, Nr. 8, S. 1420-1429) offenbart ebenfalls ein Verfahren zur in vitro Erzeugung von dreidimensionalem Knorpelgewebe. Nach diesem Verfahren werden 1 x 10⁵ oder 2 x 10⁵ Chrondrozyten für 5 Tage, 2 Wochen, 1, 2 und 3 Monate kultiviert, um Spähroide herzustellen.

Die im Stand der Technik beschriebenen in vitro hergestellten Knorpelgewebe zeigen keine Expressionsmuster essentieller Matrixproteine wie Kollagen Typ II, die denen der nativen Geweben ähnlich sind. Vielmehr weicht die Zusammensetzung der extrazellulären Matrix (EZM), die in vitro von den Chondrozyten erzeugt wird, deutlich von der nativer Knorpelgewebe ab. Die Zusammensetzung der EZM ist jedoch entscheidend für die biologische Funktionalität des Knorpels, beispielsweise die mechanische Belastbarkeit. Es besteht deshalb ein Bedarf an neuen Verfahren zur Herstellung von Knorpelgewebe, auch zur Qualitätskontrolle.

"Transplantierbares Knorpelgewebe" bedeutet eine Herstellung ex-vivo bzw. mit Hilfe eines in-vitro Verfahrens, wobei das hergestellte Gewebe weitgehend dem nativen Gewebe entspricht oder mit dem nativen Gewebe identisch ist. Im Sinne dieser Erfindung entspricht das transplantierbare Knorpelgewebe beispielsweise in Bezug auf die Expression bzw. Expressionsmuster der extrazellulären Matrix weitgehend dem des nativen Gewebes, beispielsweise in Bezug auf die Expression bzw. Expressionsmuster struktureller Proteine oder Proteide wie Kollagen Typ II und/oder S100 Protein und/oder der gewebespezifischen Proteoglykane, z.B. knorpelspezifische Proteoglykane, wie Aggrecan. Der Nachweis der Expression bzw. Expressionsmuster kann z.B. histologisch oder immunhistologisch erfolgen.

Das Gewebe, aus dem die Chondrozyten isoliert werden, ist beispielsweise ausgewählt aus muskoskelletalem Gewebe, Skelettgewebe, Knorpel, Knochen, Meniskus, Bandscheibe, Knochenmark, Gelenk oder Sehne. Das nach dem erfindungsgemäßen Verfahren hergestellte transplantierbare Knorpelgewebe ähnelt dabei dem nativen Gewebe hinsichtlich der Zusammensetzung und Proteinexpression (supra).

Natives Gelenkknorpelgewebe hat eine anteilsmäßige Zusammensetzung der extrazellulären Matrix von etwa 60 bis 80% Wasser im Bezug auf das Feuchtgewicht des Knorpelgewebes. Der hohe Wasseranteil ist wichtig für die mechanische Belastbarkeit des Knorpelgewebes und zusammen mit den Proteoglykanen für die "Schwammwirkung". Neben Wasser enthält das native Knorpelgelenk die strukturellen Proteine oder Proteine der Matrix, wie Kollagene, Proteoglykane und nicht-Kollagen Proteine. Dabei machen die strukturellen Makromoleküle etwa 20 bis 40% des Feuchtgewichts des Gelenkknorpelgewebes aus.

Bei nativem Gelenkknorpelgewebe beträgt der Kollagenanteil 60%, der Proteoglykananteil 25 bis 35% und der nicht-Kollagenprotein- und Glycoproteinanteil 15 bis 20% in Bezug auf das Knorpeltrockengewicht. Kollagen Typ II macht etwa 90 bis 95% des Gesamtkollagengehalts des nativen Gelenkknorpelgewebes aus.

Natives Gelenkknorpelgewebe des Menschen enthält etwa 1 bis 5% Knorpelzellen (Chondrozyten) in Bezug auf das Gewebevolumen. Die Knorpelzellen sind dabei die essentiellen Produzenten der Matrixmoleküle des funktionalen nativen Gewebes.

Es besteht ein hohes Bedürfnis Chondrozyten bereitzustellen, die über eine ausreichende Qualität verfügen und ein geeignetes transplantierbares Knorpelgewebe erzeugen können, die dem nativem Gewebe entspricht, so dass ein Therapieerfolg erwartet werden kann. Dieser Therapieerfolg hängt maßgeblich von der Qualität des transplantierbaren Knorpelgewebes ab.

Daher besteht die Aufgabe der Bereitstellung eines verbesserten transplantierbaren Knorpelgewebes, insbesondere mit verbesserten Eigenschaften, insbesondere hinreichender Qualität, wie Identität mit nativen Chondrozyten und qualitativ verbesserten Sphäroiden, so dass hochwertige Transplantate erhalten werden können, insbesondere zur erfolgreichen Behandlung und Therapie von Gelenkknorpeldefekten.

Überraschenderweise konnten die Erfinder Marker in isolierten Chondrozyten identifizieren, die eine hinreichende Qualität zur Herstellung von transplantierbarem Knorpelgewebe erlauben. Die erfindungsgemäßen Marker erlauben insbesondere die Bestimmung der Knorpelzellidentität ("Identitätsmarker") von den isolierten Chondrozyten in einer Kultur zu den nativen Chondrozyten.

Eine geeignete Knorpelzellidentität erlaubt wiederum die Herstellung von geeignetem transplantierbarem Knorpelgewebe, da einerseits eine hinreichende knorpelige extrazelluläre Matrix (EZM) erreicht werden kann, andererseits eine verbesserte Behandlung von Gelenkknorpeldefekten erreicht wird.

In einer weiteren Ausführungsform erlauben die erfindungsgemäßen Marker die Bestimmung der Reinheit bzw. Verunreinigung der isolierten Chondrozyten bzw. Sphäroiden ("Reinheitsmarker").

Die vorgenannten Marker gewährleisten eine hinreichende Qualität und stellen daher "Qualitätsmarker" dar.

Daher betrifft die Erfindung ein Verfahren zur Selektion von Chondrozyten oder Sphäroiden zur Herstellung von transplantierbarem Knorpelgewebe, wobei mindestens ein Qualitätsmarker bzw. Identitätsmarker oder Reinheitsmarker bestimmt wird.

Das erfindungsgemäße Verfahren zur Selektion von Chondrozyten oder Sphäroiden zur Herstellung von transplantierbarem Knorpelgewebe umfasst in einem ersten Schritt, dass die Chondrozyten in einer Monolayerkultur vermehrt werden (supra, so genannte zweidimensionale Expansionskultur), und in einem zweiten Schritt zu Sphäroiden aggregieren können (supra,so genannte 3D-Kultivierung), so dass die Chondrozyten zu Sphäroiden aggregieren, und Sphäroide ausgewählt werden können.

In einer bevorzugten Ausführungsform ist der Qualitätsmarker bzw. Identitätsmarker oder Reinheitsmarker ausgewählt aus der Gruppe KAL1, CRTAC1, NRN1 oder EBF3, welche(r) in vitro bestimmt wird (werden).

In einer bevorzugten Ausführungsform sind die Qualitätsmarker bzw. Identitätsmarker ausgewählt aus der Gruppe KAL1, CRTAC1, und/oder die Reinheitsmarker sind EBF3, NRN1. Insbesondere sind Synoviozyten nachteilige Verunreinigungen, die durch den Reinheitsmarker EBF3 in vitro bestimmt werden können. Solche Verunreinigungen reduzieren die Qualität der Chondrozyten als auch Sphäroiden.

In einer weiteren bevorzugten Ausführungsform kann zusätzlich zu den vorgenannten Markern ein so genannter Potenzialmarker erfindungsgemäß angestrengt werden, der eine Vorhersage zur Aktivität und Regenerationsfähigkeit des zu erhaltenden transplantierbaren Knorpelgewebes aus den Sphäroiden erlaubt. Solche erfindungsgemäßen Potenzialmarker sind vorzugsweise Proteoglykane, besonders bevorzugt Aggrecan (ACAN).

**Tabelle 1: Erfindungsgemäße Markergene**

| **Marker** | **Accession Nr.** | **Bezeichnung** | **Funktionen in der Qualitätstestung als auch Selektion** |
|---|---|---|---|
| CRTAC1 | NM_018058 | Homo sapiens cartilage acidic protein 1 (CRTAC1). transcript variant 1. mRNA | Chondrogener Identitätsmarker für Sphäroide |
| NRN1 | NM_001278710 | Homo sapiens neuritin 1 (NRN1). transcript variant 2. mRNA | Chondrogener Reinheitsmarker für Sphäroide |
| KAL1 (ANOS1) | NM_000216 | Homo sapiens anosmin 1 (ANOS1). mRNA | Chondrogener Identitätsmarker für |
| | | | Monolayerkultur |
| EBF3 | NM_001005463 | Homo sapiens early B-cell factor 3 (EBF3). mRNA | Chondrogener Reinheitsmarker für Sphäroide |
| ACAN | NM_001135 | Homo sapiens aggrecan (ACAN). transcript variant 1. mRNA | Potenzialmarker für Sphäroide |

Die zugehörigen DNA-Sequenzen sind über die Accession Nr. eindeutig zu identifizieren (z.B. NCBI).

Die in vitro Bestimmung der erfindungsgemäßen Marker erfolgt vorzugsweise mittels Bestimmung der Genexpression dieser Marker als auch der Level der Genexpression in den isolierten Chondrozyten als auch erhaltenden Sphäroiden, die beispielsweise routinemäßig mit der Polymerase Chain Reaction (PCR, insbesondere qPCR) festgestellt werden kann, siehe Beispiele.

Erfindungsgemäß zeigen die Marker eine Genexpression als auch erhöhte oder erniedrigte Genexpression im erfindungsgemäßen Verfahren, so dass eine Eignung als Marker vorliegt.

In einer weiteren Ausführungsform kann die Bestimmung der Genexpression bzw. Genexpressionslevel ebenfalls in Gegenwart und im Vergleich von Referenzgenen und deren Genexpression bzw. Genexpressionslevel erfolgen. Dies erlaubt eine Normalisierung der Genexpression als auch deren Quantifizierung, auch mittels Softwareunterstützung, siehe Michael W. Pfaffl, A new mathematical model for relative quantification in real-time RT-PCR, Nucleic Acids Research, 2001, Vol. 29, No. 9.

**Tabelle 2: Erfindungsgemäße Referenzgene**

| **Referenzgen** | **Accession Nr.** | **Bezeichnung** | **Funktionen in der Qualitätstestung als auch Selektion** |
|---|---|---|---|
| B2M | NM_004048 | Homo sapiens beta-2-microglobulin (B2M). mRNA | Referenzgen |
| TOP1 | NM_003286 | Homo sapiens topoisomerase (DNA) I (TOP1). mRNA | Referenzgen |

Die zugehörigen DNA-Sequenzen sind über die Accession Nr. eindeutig zu identifizieren (z.B. NCBI).

Im Rahmen dieser Erfindung bedeutet "Selektion", dass beispielsweise solche Chondrozyten oder Sphäroiden verworfen werden, die keine Genexpression der erfindungsgemäßen Marker aufweisen oder im Verhältnis zu anderen Chondrozyten oder Sphäroiden eine vergleichsweise niedrige Genexpression bzw. Genexpressionslevel aufweisen. Der Fachmann ist in der Lage anhand der erhaltenen Genexpressionsmuster eine stärkere Genexpression von einer schwächeren Genexpression zu einem oder mehreren Chondrozyten / Sphäroiden zu unterscheiden und entsprechend solche Chondrozyten oder Sphäroiden auszuwählen.

In einer weiteren bevorzugten Ausführungsform sind daher solche isolierte Chondrozyten nach Vermehrung als auch daraus erhaltene Sphäroide zu selektionieren als auch auszuwählen, die folgende Verhältnisse der Genexpression zu einem / oder beiden Referenzgenen (R) und zwar B2M und / oder TOP1 aufweisen:

| | |
|---|---|
| CRTAC1 / R | > 0,003 |
| NRN1 / R | ≥ 0,1 |
| KAL1 / R | > 1,0 |
| EBF3 / R | ≤ 0,4 |
| ACAN / R | ≥ 0,29 |

Weitere bevorzugte Verhältnisse sind solche wie

| | |
|---|---|
| CRTAC1 / R | ≥ 0,006, insbesondere ≥ 0,009 und ≥ 0,012 |
| NRN1 / R | ≥ 0,2, insbesondere ≥ 0,3 und ≥ 0,4 |
| KAL1 / R | ≥ 2,0, insbesondere ≥ 3,0 und ≥ 4,0 |
| EBF3 / R | ≤ 0,3, insbesondere ≤ 0,2 und ≤ 0,1 |
| ACAN / R | ≥ 0,39, insbesondere ≥ 0,49 und ≥ 0,59 |

Die angegebenen Werte verstehen sich als geeignete Schwellenwerte.

Daher betrifft die Erfindung ein Verfahren zur Selektion von Chondrozyten oder Sphäroiden zur Herstellung von transplantierbarem Knorpelgewebe, wobei im Vergleich zu einem Referenzgen die erfindungsgemäßen Marker bestimmt werden.

In einer weiteren bevorzugten Ausführungsform ist im Zuge der duchgeführten Bestimmungen des Reinheitsmarkers EBF3 der Anteil an Synoviozyten als Verunreinigung in den isolierten Chondrozyten nach Vermehrung oder in den erhaltenen Sphäroiden im Verhältnis zur Gesamtanzahl der Zellen weniger oder gleich 9 %, insbesondere weniger oder gleich 5 %.

Das erfindungsgemäße Verfahren erlaubt die Selektion von Chondrozyten als auch Sphäroiden, die spezifisch für ein zu transplantierbares Knorpelgewebe ausgewählt werden. Dies liegt an den besonderen Eigenschaften der erfindungsgemäßen Marker, nämlich Qualitätsmarker bzw. Identitätsmarker oder Reinheitsmarker. Folglich werden solche Chondrozyten als auch Sphäroiden ausgewählt, die eine spezifische Genexpression aufweisen und entsprechende Genprodukte zum Beispiel mittels PCR nachgewiesen werden können.

Daher betrifft die Erfindung isolierte Chondrozyten oder isolierte erhaltene Sphäroiden, erhältlich durch ein erfindungsgemäßes Verfahren. Weiterhin umfasst ist ein transplantierbares Knorpelgewebe, insbesondere in Form eines Mittels bzw. Arzneimittels oder in Form einer pharmazeutischen Zusammensetzung enthaltend solche Chondrozyten oder Sphäroiden, insbesondere zur Verwendung in der Behandlung von Gelenkknorpeldefekten und / oder autologen Chondrozytentransplantation.

In einer weiteren bevorzugten Ausführungsform der Erfindung wird das erfindungsgemäße transplantierbare Knorpelgewebe mit Hilfe eines Applikators (siehe EP2345450B1 (co.fix 150 der Anmelderin) oder DE102009025347A1) einem Patienten verabreicht. Vorzugsweise kann das transplantierte Knorpelgewebe in Form einer pharmazeutischen Zusammensetzung in einer physiologischen Kochsalzlösung samt weiteren Hilfs-, Zusatzstoffen gehalten werden.

Die nachfolgenden Figuren und Beispiele dienen der Erläuterung der Erfindung ohne die Erfindung jedoch auf die Figuren und Beispiele einzuschränken.

### Beispiel 1:

### Isolierung von Knorpelzellen (Chondrozyten) aus Knorpelbiopsie und Zellkultivierung

Knorpelspezifische Zellen (Chondrozyten) wurden aus einer Knorpelbiopsie mittels enzymatischem Verdau unter Verwendung von Kollagenase (350 Einheiten/ml) isoliert. Der enzymatische Verdau dauerte zwischen 4 und 6 Stunden und fand bei 37 °C statt. Die Knorpelbiopsie wurde aus der femuralen Kondyle des Kniegelenks entnommen. Nach dem enzymatischen Verdau wurden die Chondrozyten zentrifugiert, gewaschen, gezählt und in Gewebekulturflaschen zusammen mit Zellkulturmedium (Alpha MEM/ Ham's F12 1: 1, 1% Glutamin) und autologem Serum (10-15% Humanserum) ausgesät. Zur Expansion wurden die Zellen in vitro zwischen 7 und 38 Tagen bei 37 °C in der zweidimensionalen Expansionskultur (Monolayer) kultiviert. Der Austausch von Zellkulturmedium fand alle 3-6 Tage statt. Wenn die Zellkulturen konfluent wurden, wurde die Zellkultur mithilfe von Papain passagiert und über mehrere Kulturflaschen aufgeteilt.

Nach Erreichen einer ausreichenden Anzahl von Zellen aus der Monolayer-Kultur wurden je 200.000 Zellen pro Well in Mikrotiterplatten, die mit 2% Agarose beschichtet sind, ausgesät. Die Zellen aggregieren und bilden eine dreidimensionale, runde Struktur (Sphäroid). Während der Kultivierung der Sphäroide wurde Zellkulturmedium (Alpha MEM/ Ham's F12 1: 1; 1% Glutamin), welches mit 10% autologen Serum versetzt wurde, regelmäßig ausgetauscht (alle 3-6 Tage). Die 3D-Kultivierung dauerte zwischen 14 und 42 Tagen.

Nach der Sphäroidkultivierung wurde das Knorpelzelltransplantat für den Transport und die Implantation vorbereitet, indem man die Sphäroide in den Arzneimittelträger (0,9% NaCl) sammelte und anschließend in das Applikatorsystem co.fix 150 oder eine Spritze überführte.

Zusammengefasst besteht die Herstellung von Knorpelzelltransplantaten aus einer Monolayer-Kultivierungsphase, um die isolierten Chondrozyten zu vermehren und einer 3D-Kultivierungsphase um die Chondrozyten zu Sphäroiden zu aggregieren. Das Knorpelzelltransplantat ist für die autologe Anwendung, sprich für die Implantation im Knie desselben Patienten bestimmt.

### Beispiel 2:

### a.) RNA-Isolierung

Die Knorpelzellen wurden bis zur Herstellung der Sphäroide in einer zweidimensionalen Expansionskultur (Monolayer) kultiviert und anschließend mithilfe einer Enzymlösung (Papain) vom Boden der Zellkulturflasche und aus dem Zellverband gelöst. 1x10E6 dieser Knorpelzellen wurden für die Identitätsprüfung aus der Zellsuspension entnommen und in ein Reaktionsgefäß überführt, während die restlichen Zellen für die darauffolgende Herstellung der Sphäroide genutzt wurden. Die prüfungsrelevanten Monolayer-Zellen wurden abzentrifugiert und das Zellpellet im entsprechenden Lysepuffer (PeqGOLD, Peqlab) lysiert, schockgefroren und bis zur RNA-Isolierung bei -70 °C gelagert. Für die Markertestung an Knorpelzell-Sphäroiden wurden nach zweiwöchiger Kulturdauer 8 Sphäroide aus der Well-Platte entnommen, mit PBS gewaschen und mit dem entsprechenden Lysepuffer (PeqGOLD, Peqlab) lysiert. Die im Lysepuffer aufgenommenen Sphäroide wurden für einen optimalen Zellaufschluss mittels Spritze und Kanüle mechanisch homogenisiert, indem die Sphäroide auf- und abgezogen wurden. Daraufhin wurde das Lysat in flüssigem Stickstoff schockgefroren und bis zur RNA-Isolierung bei -70 °C gelagert.

Die Extraktion der Gesamt-RNA aus den Knorpelzell-Sphäroiden und -Monolayerzellen erfolgte anhand des peqGOLD Total RNA Kits der Firma Peqlab oder mit dem RNeasy Plus Mini Kit der Firma Qiagen gemäß den Anweisungen des Herstellers. Die isolierte RNA wurde in nukleasefreiem Wasser eluiert und bis zur cDNA-Synthese bei -70 °C gelagert. Nach der RNA-Isolierung wurde die Quantität, Qualität und Integrität der RNA mit einem Spektrophotometer (NanoDrop, Thermo Scientific) und mittels eines Bioanalyzers (Agilent) nach den Anweisungen der Hersteller bestimmt.

### b.) cDNA-Synthese

Die cDNA-Synthese erfolgte anhand des Transcriptor First Strand cDNA Synthesis Kit (Roche) gemäß den Anweisungen des Herstellers. Für jede Probe wurden 80 ng Gesamt-RNA unter Verwendung der random Hexamer-Primer transkribiert. In einem sterilen Reaktionsgefäß wurde der Template-Primer-Mix für ein Reaktionsvolumen von 20 µl angesetzt. Zum Template-Primer-Mix wurden folgende weiteren Komponenten hinzupipettiert: Transcriptor Reverse Transcriptase Reaction Buffer, Protector RNase Inhibitor, Deoxynucleotide Mix, Transcriptor Reverse Transcriptase. Die Reaktionsansätze wurden gemixt, herunterzentrifugiert und anschließend im Thermocycler inkubiert. Die revers-transkribierte cDNA wurde anschließend mit nukleasefreiem Wasser bis zu einer Endkonzentration von 1 ng/µl verdünnt. Die Proben wurden bis zur weiteren Verwendung in den qPCR-Analysen bei -20 °C gelagert.

### c.) qPCR

Für die qPCR-Analysen wurde die qPCR-Technik mit dem LightCycler® 480 Instrument II (Roche) im 384-Well-Format genutzt. Die Primer waren HPLC gereinigt und wurden in nukleasefreiem Wasser auf eine Konzentration von 10 µM gelöst. Die Sonden wurden in einer Konzentration von 10 µM eingesetzt. Für die zu messenden Marker wurden zuvor definierte qPCR-Reaktionsbedingungen bezüglich der Primer- und Sondenkonzentration sowie für die Verwendung von Additiven etabliert. Für alle Proben wurde eine Mastermischung gemäß der Reaktionsbedingung der spezifischen Primer hergestellt. Jeweils 8 µl des für jedes Zielgen spezifischen Reaktionsmixes wurde in die entsprechende Vertiefung der 384-Well-Platte pipettiert und anschließend 2 µl cDNA-Probe (1 ng/µl) hinzugegeben, womit sich pro qPCR-Reaktion ein Gesamtvolumen von 10 µl ergab.

Die qPCR-Analyse basiert auf der exponentiellen Zunahme der fluoreszenz-markierten PCR-Amplifikate des Gens von Interesse. Die Auswertung der qPCR-Daten erfolgte mit Hilfe der LightCycler® 480 Software unter Verwendung des Softwaremoduls Advanced Relative Quantification (Roche). Das verwendete Detektionsformat war das sogenannte Mono Color Hydrolyse Probe/UPL Probe Programm mit den folgenden Parametern: Anregungsfilter 483 nm, Emissionsfilter 533 nm, Filterkombination FAM. qPCR-Daten wurden mit den Softwaremodulen zur relativen Quantifizierung erzeugt und analysiert. Absolute qPCR-Daten (CP-Werte) der jeweiligen Zielgene wurden mit der Methode Abs Quant/2nd Derivative Max erzeugt. Der Cp-Wert (Crossing point oder Zyklusschwelle) ist die Anzahl an PCR-Zyklen, die erforderlich sind, um einen definierten Schwellenwert des Fluoreszenzsignals zu überschreiten. Die Fluoreszenz-Signalerfassung wurde am Ende eines jeden Zyklus bei 72 °C durchgeführt.

Die Normalisierung der mRNA-Expressionslevel der Zielgene gegenüber Referenzgene wurde mit Hilfe der sogenannten E-(Efficiency)-Methode unter Verwendung des Softwaremoduls Advanced Relative Quantification (Roche) durchgeführt. Zwei Referenzgene, TOP1 und B2M, wurden jeweils als interne Normalisierungskontrolle für jede Probe mitgemessen. Die Expression der Zielgene wurde auf diese Referenzgene normiert und normalisierte Daten wurden als Ziel/Referenz-Werte (T/R) automatisch vom Softwaremodul Advanced Relative Quantification bereitgestellt. Das diesem Softwaremodul zugrundeliegende mathematische Modell basiert auf der Beziehung zwischen den Cp-Werten des Zielgens im Vergleich zu den Referenzgenen und berücksichtigt u.a. die primerspezifische Amplifikationseffizienz.

Die normalisierten Expressionswerte werden für die Prüfung der Qualität der Knorpelzelltransplantate benutzt. Kritische Qualitätsattribute stellen Identität, Reinheit und Potenzial dar, wobei für jeden Qualitätsparameter mindestens ein spezifischer Marker identifiziert wurde, der zum Nachweis ausreichender Qualität gemessen werden kann. Die normalisierten Expressionswerte eines Markers müssen dabei für den Nachweis ausreichender Qualität definierte Akzeptanzgrenzen erfüllen. Der Wert für die Qualitätsparameter basiert auf den mRNA-Expressionsniveaus in Monolayer-Knorpelzellen (KAL1) oder -Sphäroiden (CRTAC1, NRN1, EBF3 und ACAN).

Tabelle 3 zeigt die Anwendung der beschriebenen Selektion auf Knorpeltransplantatprodukte. Insgesamt wurden 48 Knorpeltransplantatprodukte wie beschrieben hergestellt und einer Prüfung auf Identität, Reinheit (Verunreinigung) als auch Potential mit den erfindungsgemäßen Markern unterzogen. Achtundvierzig der 48 Knorpeltransplantate erfüllten die Kriterien der Identität im Monolayer (KAL1) und im Sphäroid (CRTAC1). Vier der 48 Knorpeltransplantate erfüllten nicht das Kriterium Reinheit(Verunreinigung) (EBF3, NRN1) und 19 der 48 Knorpeltransplantate erfüllten nicht das Kriterium des Potentials. Insgesamt erfüllten 22 der 48 Knorpelzelltransplantate nicht das Kriterium der Knorpelzellqualität (Identität, Reinheit/Verunreinigung oder Potential). Sechsundzwanzig der 48 Knorpelzelltransplantate erfüllten das Kriterium der Knorpelzellqualität (Identität, Reinheit/Verunreinigung oder Potential) und sind zur Herstellung von transplantierbarem Knorpelgewebe geeignet und wurden ausgewählt.

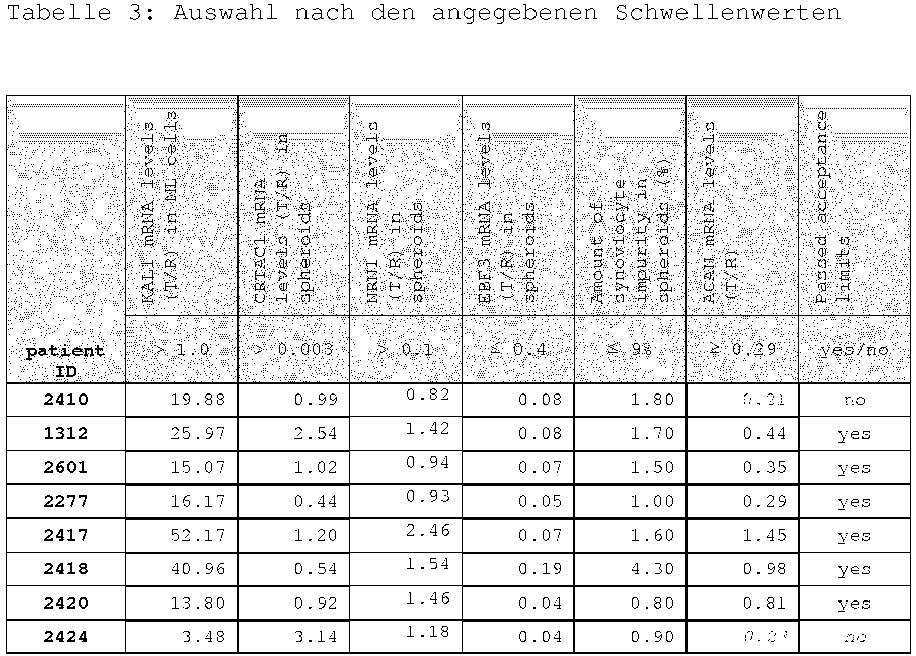

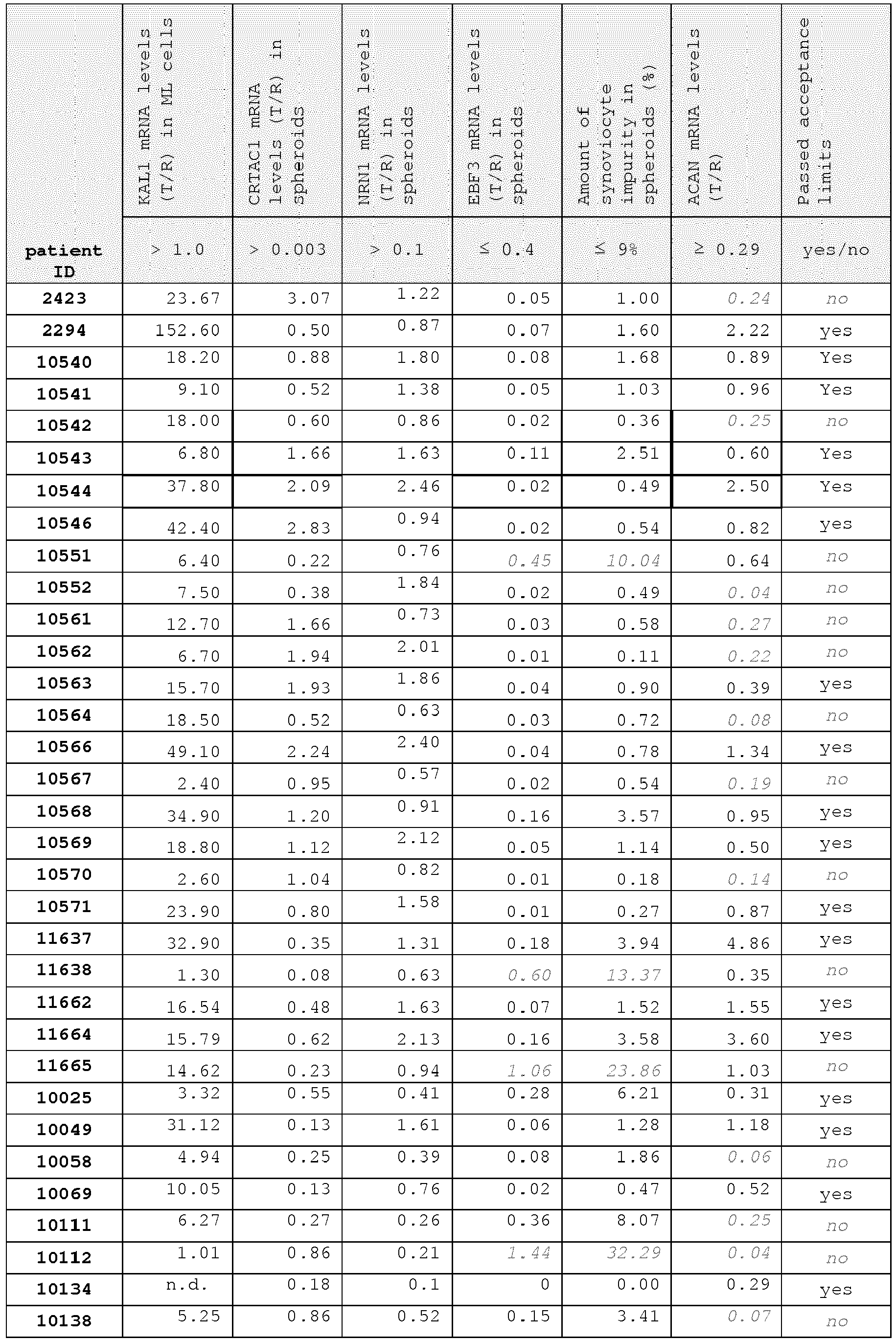

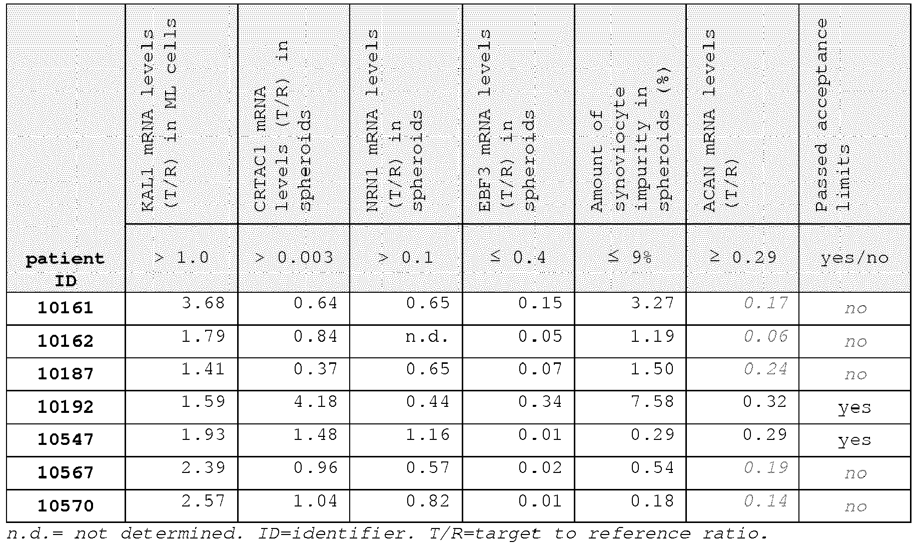

Beispiel 3: Herstellung von zu transplantierendem Knorpelgewebe mittels in Monolayerkultur vermehrter Knorpelzellen definierter Qualität, hier: Identität Vom Patienten mit zu behandelndem Knorpeldefekt wird aus einem gesunden Knorpelareal eine Biopsie hyalinen Knorpels entnommen. Durch enzymatischen Verdau des Knorpelgewebes mit einer Kollagenaselösung werden die im Knorpelgewebe befindlichen Knorpelzellen (Chondrozyten) isoliert und mittels Standardzellkulturbedingungen in Zellkulturflaschen (Monolayerkultur) in der Gegenwart von autologem Serum vermehrt, so dass ein maximaler Population Doubling Level (PDL) von 10 nicht überschritten wird und ausreichend Chondrozyten zur Herstellung von transplantierbarem Knorpelgewebe zur Verfügung stehen. Ein Teil der im Monolayer vermehrten Knorpelzellen wird zur Bestimmung der Knorpelzellidentität und der übrige Teil zur Herstellung von transplantierbarem Knorpelgewebe verwendet.

Der Marker zur Knorpelzellidentität in der Monolayerkultur zeigt an, dass Knorpelzellen in der Knorpelzellkultur vorliegen. Zur Knorpelzellidentität in der Monolayerkultur wird mittels gängiger molekularbiologischer Techniken die Gesamt-RNA der im Monolayer kultivierten Chondrozyten isoliert und mittels reverser Transkriptase in cDNA umgeschrieben. Mittels gängiger Polymerase-Kettenreaktion (PCR) wird die Expression wenigstens eines Referenzgens (supra, Tabelle 2) und des Identitätsmarkers "KAL1" bestimmt. Die Expression von "KAL1" wird in Relation zu der Expression des Referenzgens gesetzt und ergibt so einen normalisierten Genexpressionswert für den Identitätsmarker "KAL1". Die Expression von "KAL1" zeigt an, dass Chondrozyten ausreichender Qualität (Identität) zur Herstellung von transplantierbarem Knorpelgewebe vorliegen.

Zur Herstellung der transplantierbaren Knorpelgewebe werden wenigstens 100.000 der im Monolayer vermehrten Chondrozyten ausreichender Qualität (Identität) in ein Zellkulturgefäß in der Gegenwart von Standardzellkulturmedien und autologem Serum überführt. Das Zellkulturgefäß verhindert mittels zum Beispiel einer Agarosebeschichtung des Zellkulturbodens ein Anheften der Chondrozyten. Nach wenigen Tagen haften die Chondrozyten aneinander und bilden Aggregate, sogenannte Sphäroide aus. Die Sphäroide werden über einen Zeitraum von wenigstens 1-2 Wochen kultiviert und bilden eine knorpelige extrazelluläre Matrix aus (Sphäroidkultur). Dieses Knorpelgewebe (Chondrozyten ausreichender Qualität (Identität) und knorpelige extrazelluläre Matrix) ist geeignet, um nach Einbringen in den Knorpeldefekt des zu behandelnden Patienten ein Knorpelersatzgewebe aufzubauen, welches den Defekt füllt und die Funktion des natürlichen Knorpelgewebes übernimmt. Knorpelgewebe unzureichender Qualität (Identität in der Monolayerkultur) ist nicht zum Aufbau von Knorpelersatzgewebe geeignet und wird verworfen.

Beispiel 4: Herstellung von zu transplantierendem Knorpelgewebe mittels in Sphäroidkultur befindlicher Knorpelzellen definierter Qualität, hier: Identität Die in Beispiel 3 isolierten und im Monolayer vermehrten Knorpelzellen werden zur Herstellung von transplantierbarem Knorpelgewebe verwendet.

Wie in Beispiel 3 beschrieben, werden die im Monolayer vermehrten Chondrozyten in ein spezielles Zellkulturgefäß überführt, welches ein Anheften der Chondrozyten verhindert, so dass sich nach wenigen Tagen Aggregate, sogenannte Sphäroide, ausbilden. Die Sphäroide werden über einen Zeitraum von wenigstens 1-2 Wochen kultiviert und bilden eine knorpelige extrazelluläre Matrix aus (Sphäroidkultur). Der Marker zur Knorpelzellidentität in der Sphäroidkultur zeigt an, dass Knorpelzellen in der Sphäroidkultur vorliegen. Zur Bestimmung der Knorpelzellidentität in der Sphäroidkultur wird mittels gängiger molekularbiologischer Techniken die Gesamt-RNA eines Teiles der in Sphäroiden kultivierten Chondrozyten isoliert und mittels reverser Transkriptase in cDNA umgeschrieben. Mittels gängiger Polymerase-Kettenreaktion (PCR) wird die Expression wenigstens eines Referenzgens (supra, Tabelle 2) und des Identitätsmarkers "CRTAC1" bestimmt. Die Expression von "CRTAC1" wird in Relation zu der Expression des Referenzgens gesetzt und ergibt so einen normalisierten Genexpressionswert für den Identitätsmarker "CRTAC1". Die Expression von "CRTAC1" zeigt an, dass Chondrozyten ausreichender Qualität (Identität) im Sphäroid zur Transplantation des Knorpelgewebes vorliegen.

Dieses Knorpelgewebe (Chondrozyten ausreichender Qualität (Identität) und knorpelige extrazelluläre Matrix) ist geeignet, um nach Einbringen in den Knorpeldefekt des zu behandelnden Patienten ein Knorpelersatzgewebe aufzubauen, welches den Defekt füllt und die Funktion des natürlichen Knorpelgewebes übernimmt. Knorpelgewebe unzureichender Qualität (Identität in der Sphäroidkultur) ist nicht zum Aufbau von Knorpelersatzgewebe geeignet und wird verworfen.

Beispiel 5: Herstellung von zu transplantierendem Knorpelgewebe mittels in Sphäroidkultur befindlicher Knorpelzellen definierter Qualität, hier: Reinheit Die in Beispiel 3 isolierten und im Monolayer vermehrten Knorpelzellen werden zur Herstellung von transplantierbarem Knorpelgewebe verwendet.

Wie in Beispiel 3 beschrieben, werden die im Monolayer vermehrten Chondrozyten in ein spezielles Zellkulturgefäß überführt, welches ein Anheften der Chondrozyten verhindert, so dass sich nach wenigen Tagen Aggregate, sogenannte Sphäroide, ausbilden. Die Sphäroide werden über einen Zeitraum von wenigstens 1-2 Wochen kultiviert und bilden eine knorpelige extrazelluläre Matrix aus (Sphäroidkultur).

Der Marker zur Knorpelzellreinheit zeigt den Gehalt an Knorpelzellen in der Sphäroidkultur an. Zur Bestimmung der Knorpelzellreinheit in der Sphäroidkultur wird mittels gängiger molekularbiologischer Techniken die Gesamt-RNA eines Teiles der in Sphäroiden kultivierten Chondrozyten isoliert und mittels reverser Transkriptase in cDNA umgeschrieben. Mittels gängiger Polymerase-Kettenreaktion (PCR) wird die Expression wenigstens eines Referenzgens (supra, Tabelle 2) und des Reinheitsmarkers "NRN1" bestimmt. Die Expression von "NRN1" wird in Relation zu der Expression des Referenzgens gesetzt und ergibt so einen normalisierten Genexpressionswert für den Reinheitsmarker "NRN1". Übersteigt die Expressionshöhe von "NRN1" den zuvor definierten Schwellenwert für die Reinheit, z.B. 1.0, zeigt dieses an, dass Chondrozyten ausreichender Qualität (Reinheit) im Sphäroid zur Transplantation des Knorpelgewebes vorliegen. Ist der Schwellenwert für die Reinheit unterschritten, liegen Chondrozyten unzureichender Qualität (Reinheit) im Sphäroid zur Transplantation des Knorpelgewebes vor.

Dieses Knorpelgewebe (Chondrozyten ausreichender Qualität (Reinheit) und knorpelige extrazelluläre Matrix) ist geeignet, um nach Einbringen in den Knorpeldefekt des zu behandelnden Patienten ein Knorpelersatzgewebe aufzubauen, welches den Defekt füllt und die Funktion des natürlichen Knorpelgewebes übernimmt. Knorpelgewebe unzureichender Qualität (Erniedrigter Reinheitsgrad) ist nicht zum Aufbau von Knorpelersatzgewebe geeignet und wird verworfen.

Beispiel 6: Herstellung von zu transplantierendem Knorpelgewebe mittels in Sphäroidkultur befindlicher Knorpelzellen und definierter zellulärer Verunreinigungen Die in Beispiel 3 isolierten und im Monolayer vermehrten Knorpelzellen werden zur Herstellung von transplantierbarem Knorpelgewebe verwendet.

Wie in Beispiel 3 beschrieben, werden Sphäroide gebildet und über einen Zeitraum von wenigstens 1-2 Wochen kultiviert, um eine knorpelige extrazelluläre Matrix auszubilden (Sphäroidkultur).

Der Marker zur Knorpelzellverunreinigung zeigt den Gehalt an zellulären Verunreinigungen mit Synovialzellen, Knochen- und/oder Fettzellen in der Sphäroidkultur an. Zur Bestimmung von zellulären Verunreinigungen der Knorpelzellen in Sphäroidkultur wird mittels gängiger molekularbiologischer Techniken die Gesamt-RNA eines Teiles der in Sphäroiden kultivierten Chondrozyten isoliert und mittels reverser Transkriptase in cDNA umgeschrieben. Mittels gängiger Polymerase-Kettenreaktion (PCR) wird die Expression wenigstens eines Referenzgens (supra, Tabelle 2) und des Verunreinigungsmarkers "EBF3" bestimmt. Die Expression von "EBF3" wird in Relation zu der Expression des Referenzgens gesetzt und ergibt so einen normalisierten Genexpressionswert für den Verunreinigungsmarker "EBF3". Unterschreitet die Expressionshöhe von "EBF3" den zuvor definierten Schwellenwert für die Verunreinigung, z.B. 0.8, zeigt dieses an, dass keine übermäßigen zellulären Verunreinigungen durch Synoviozyten im Sphäroid zur Transplantation des Knorpelgewebes vorliegen.. Ist der Schwellenwert für die Verunreinigung überschritten, liegen Chondrozyten unzureichender Qualität (Verunreinigung) im Sphäroid zur Transplantation des Knorpelgewebes vor. Dieses Knorpelgewebe (Chondrozyten ausreichende Qualität (Verunreinigung) und knorpelige extrazelluläre Matrix) ist geeignet, um nach Einbringen in den Knorpeldefekt des zu behandelnden Patienten ein Knorpelersatzgewebe aufzubauen, welches den Defekt füllt und die Funktion des natürlichen Knorpelgewebes übernimmt. Knorpelgewebe unzureichender Qualität (Erhöhter Gehalt an zellulären Verunreinigungen) ist nicht zum Aufbau von Knorpelersatzgewebe geeignet und wird verworfen.

Beispiel 7: Herstellung von zu transplantierendem Knorpelgewebe mittels in Sphäroidkultur befindlicher Knorpelzellen definierten Potentials Die in Beispiel 3 isolierten und im Monolayer vermehrten Knorpelzellen werden zur Herstellung von transplantierbarem Knorpelgewebe verwendet.

Wie in Beispiel 3 beschrieben, werden Sphäroide gebildet und über einen Zeitraum von wenigstens 1-2 Wochen kultiviert, um eine knorpelige extrazelluläre Matrix auszubilden (Sphäroidkultur).

Der Marker zum Knorpelzellpotential zeigt an, dass Knorpelzellen in der Sphäroidkultur die Fähigkeit aufweisen knorpelige extrazelluläre Matrix zu bilden. Zur Bestimmung des Potentials von Knorpelzellen in Sphäroidkultur wird mittels gängiger molekularbiologischer Techniken die Gesamt-RNA eines Teiles der in Sphäroiden kultivierten Chondrozyten isoliert und mittels reverser Transkriptase in cDNA umgeschrieben. Mittels gängiger Polymerase-Kettenreaktion (PCR) wird die Expression wenigstens eines Referenzgens und des Potentialmarkers "Aggrecan" bestimmt. Die Expression von "Aggrecan" wird in Relation zu der Expression des Referenzgens gesetzt und ergibt so einen normalisierten Genexpressionswert für den Potentialmarker "Aggrecan". Überschreitet die Expressionshöhe von "Aggrecan" den zuvor definierten Schwellenwert für das Potential, z.B. 0.1, zeigt dieses an, dass Chondrozyten ausreichender Qualität (Potential) im Sphäroid zur Transplantation des Knorpelgewebes vorliegen. Ist der Schwellenwert für das Potential unterschritten liegen Chondrozyten unzureichender Qualität (unzureichendes Potential knorpelige extrazelluläre Matrix zu bilden) im Sphäroid zur Transplantation des Knorpelgewebes vor.

Dieses Knorpelgewebe (Chondrozyten ausreichende Qualität (Potential) und knorpelige extrazelluläre Matrix) ist geeignet, um nach Einbringen in den Knorpeldefekt des zu behandelnden Patienten ein Knorpelersatzgewebe aufzubauen, welches den Defekt füllt und die Funktion des natürlichen Knorpelgewebes übernimmt. Knorpelgewebe unzureichender Qualität (Potential) ist nicht zum Aufbau von Knorpelersatzgewebe geeignet und wird verworfen.

Beispiel 8: Herstellung von zu transplantierendem Knorpelgewebe mittels Knorpelzellen definierter Qualität Die in Beispiel 3 isolierten und im Monolayer vermehrten Knorpelzellen werden zur Herstellung von transplantierbarem Knorpelgewebe verwendet.

Wie in Beispiel 3 beschrieben, werden Sphäroide gebildet und über einen Zeitraum von wenigstens 1-2 Wochen kultiviert, um eine knorpelige extrazelluläre Matrix auszubilden (Sphäroidkultur).

Zum Nachweis qualitativ hochwertiger Sphäroidkulturen zur Transplantation und zur Regeneration von artikulären Knorpeldefekten wird, wie in Beispiel 3 beschrieben, die Identität mittels des Identitätsmarkers "KAL1" und mittels des Identitätsmarkers "CRTAC1", wie in Beispiel 4 beschrieben, bestimmt. Zudem wird, wie in Beispiel 5 beschrieben, der Reinheitsmarker "NRN1" und/oder, wie in Beispiel 6 beschrieben, der Verunreinigungsmarker "EBF3" bestimmt. Weiterhin wird, wie in Beispiel 7 beschrieben, der Potentialmarker "Aggrecan" bestimmt.

Knorpelgewebe (Chondrozyten ausreichender Qualität (Identität in der Monolayerkultur und/oder in der Sphäroidkultur, ausreichende Reinheit und/oder akzeptabler Grad an zellulären Verunreinigungen sowie ausreichendes Potential knorpelige extrazelluläre Matrix zu bilden) und knorpelige extrazelluläre Matrix) ist geeignet, um nach Einbringen in den Knorpeldefekt des zu behandelnden Patienten ein Knorpelersatzgewebe aufzubauen, welches den Defekt füllt und die Funktion des natürlichen Knorpelgewebes übernimmt. Knorpelgewebe unzureichender Qualität (Identität in der Monolayerkultur und/oder in der Sphäroidkultur nicht gegeben, unzureichende Reinheit und/oder unzureichender Grad an zellulären Verunreinigungen sowie unzureichendes Potential knorpelige extrazelluläre Matrix zu bilden) ist nicht zum Aufbau von Knorpelersatzgewebe geeignet und wird verworfen.

Beispiel 9: Retrospektiv wurde das hier in Beispiel 8 beschriebene Vorgehen auf zu transplantierende Knorpelgewebe von 10 Patienten angewendet, bei denen Knorpelgewebe zur Regeneration von Kniegelenkknorpelschäden verwendet wurde. Die Fähigkeit des transplantierten Knorpelgewebes Knorpelersatzgewebe aufzubauen, wurde klinisch anhand eines etablierten Knorpelreparaturscores (KOOS - Knee injury and Osteoarthritis Outcome Score: Roos EM & Lohmander LS, Health and Quality of Life Outcomes 2003, I:64), sprich dem klinischen Erfolg der Behandlung beurteilt. Hierzu wurde mittels Patientenbefragung der KOOS des jeweiligen Patienten vor der Behandlung mit dem zu transplantierendem Knorpelgewebe bestimmt (Basiswert). Ein Jahr nach der Behandlung mit dem zu transplantierendem Knorpelgewebe wurde erneut mittels Patientenbefragung der KOOS des jeweiligen Patienten bestimmt (1 Jahr Nachbeobachtungswert). Der klinische oder therapeutische Erfolg der Behandlung ist gegeben, wenn die Differenz (KOOS delta) zwischen 1 Jahr Nachbeobachtungswert und Basiswert wenigstens 8 Punkte beträgt (Roos EM & Lohmander LS, Health and Quality of Life Outcomes 2003, I:64). Ist die Differenz (KOOS delta) zwischen 1 Jahr Nachbeobachtungswert und Basiswert geringer als 8 Punkte liegt keine klinische Verbesserung durch die Behandlung vor, d.h. die Therapie ist nicht erfolgreich gewesen.

Es zeigt sich, dass beim transplantierbaren Knorpelgewebe von 4 der 10 Patienten (5697 - 1607, 5862 - 1311, 6070 - 2413, 6988 - 2423) der Potentialmarker "Aggrecan" unterhalb des festgelegten Grenzwertes liegt, während die Identität der Knorpelzellen anhand der Identitätsmarker "KAL1" bzw. "CRTAC1" festgestellt werden konnte. Die Expressionswerte liegen oberhalb der festgelegten Grenzwerte. Auch die Reinheit der zu transplantierenden Knorpelgewebe kann anhand des Verunreinigungsmarkers "EBF3" bestimmt werden und ist gegeben; hier liegen Expressionswerte unterhalb der festgelegten Grenzwerte.

Beim transplantierbaren Knorpelgewebe von 6 der 10 Patienten (6266 - 2601, 6658 - 2420, 7494 - 2284, 8528 - 2286, 9070 - 2709, 9110 - 2294) liegt der Potentialmarker "Aggrecan" oberhalb des festgelegten Grenzwertes, und die Identität der Knorpelzellen kann anhand der Identitätsmarker "KAL1" bzw. "CRTAC1" festgestellt werden. Die Expressionswerte liegen ebenfalls oberhalb der festgelegten Schwellenwerte. Auch die Reinheit der zu transplantierenden Knorpelgewebe konnte anhand des Verunreinigungsmarkers "EBF3" bestimmt werden und ist gegeben; hier liegen die Expressionswerte unterhalb der festgelegten Schwellenwerte.

Demnach zeigen die transplantierbaren Knorpelgewebe von 4 der 10 Patienten (5697 - 1607, 5862 - 1311, 6070 - 2413, 6988 - 2423) ein Knorpelgewebe unzureichender Qualität (Identität in der Monolayerkultur und/oder in der Sphäroidkultur nicht gegeben, unzureichende Reinheit und/oder unzureichender Grad an zellulären Verunreinigungen sowie unzureichendes Potential knorpelige extrazelluläre Matrix zu bilden), während die transplantierbaren Knorpelgewebe von 6 der 10 Patienten (6266 - 2601, 6658 - 2420, 7494 - 2284, 8528 - 2286, 9070 - 2709, 9110 - 2294) ein Knorpelgewebe ausreichender Qualität aufweisen (Identität in der Monolayerkultur und/oder in der Sphäroidkultur gegeben, ausreichende Reinheit und/oder ausreichender Grad an zellulären Verunreinigungen sowie ausreichendes Potential knorpelige extrazelluläre Matrix zu bilden).

Bezüglich des klinischen oder therapeutischen Erfolges der Behandlung zeigt sich (siehe Tabelle 4), dass bei denjenigen 4 der 10 Patienten (5697 - 1607, 5862 - 1311, 6070 - 2413, 6988 - 2423), bei denen retrospektiv ein transplantierbares Knorpelgewebe unzureichender Qualität festgestellt wurde, keine klinische Verbesserung durch die Behandlung vorliegt. Die Differenz zwischen dem KOOS 1 Jahr Nachbeobachtungswert und dem KOOS Basiswert war geringer als 8 Punkte, die Therapie war folglich nicht erfolgreich. Demgegenüber zeigt sich, dass bei denjenigen 6 der 10 Patienten (6266 - 2601, 6658 - 2420, 7494 - 2284, 8528 - 2286, 9070 - 2709, 9110 - 2294), bei denen retrospektiv ein transplantierbares Knorpelgewebe ausreichender Qualität festgestellt wurde, eine klinische Verbesserung durch die Behandlung vorliegt. Die Differenz zwischen dem KOOS 1 Jahr Nachbeobachtungswert und dem KOOS Basiswert beträgt wenigstens 8 Punkte, die Therapie ist erfolgreich gewesen.

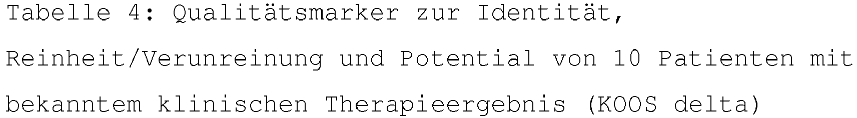

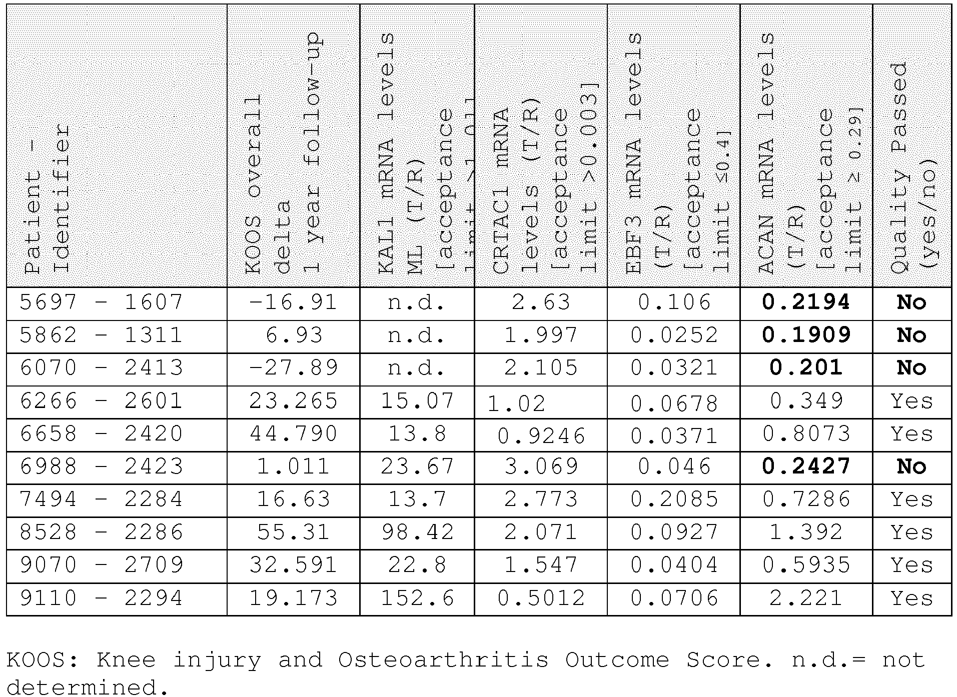

## Patentansprüche

1. Verfahren zur Selektion von Chondrozyten zur Herstellung von transplantierbarem Knorpelgewebe, **dadurch gekennzeichnet, dass** mindestens ein Identitätsmarker oder Reinheitsmarker, ausgewählt aus der Gruppe KAL1, CRTAC1, NRN1 oder EBF3, in vitro bestimmt wird.

2. Verfahren zur Selektion von Chondrozyten nach Patentanspruch 1, **dadurch gekennzeichnet, dass** zur Herstellung von transplantierbarem Knorpelgewebe die Chondrozyten in einem ersten Schritt in einer Monolayerkultur vermehrt werden, und in einem zweiten Schritt zu Sphäroiden aggregieren.

3. Verfahren zur Selektion von Chondrozyten nach einem der vorstehenden Patentansprüche 1 oder 2, **dadurch gekennzeichnet, dass** die Chondrozyten zu Sphäroiden aggregiert sind, und Sphäroide ausgewählt werden.

4. Verfahren zur Selektion von Chondrozyten nach einem der vorstehenden Patentansprüche, **dadurch gekennzeichnet, dass** die Identitätsmarker ausgewählt sind aus der Gruppe KAL1, CRTAC1 und/oder, dass die Reinheitsmarker ausgewählt sind aus der Gruppe NRN1, EBF3.

5. Verfahren zur Selektion von Chondrozyten nach einem der vorstehenden Patentansprüche, **dadurch gekennzeichnet, dass** i.) Chondrozyten in einer Monolayerkultur mit dem Identitätsmarker KAL1 in vitro bestimmt wird und / oder ii.) Chondrozyten in Sphäroiden mit mindestens einem Identitätsmarker CRTAC1 in-vitro bestimmt wird.

6. Verfahren zur Selektion von Chondrozyten nach einem der vorstehenden Patentansprüche, **dadurch gekennzeichnet, dass** zusätzlich Proteoglykane, insbesondere Aggrecan in-vitro bestimmt werden.

7. Verfahren zur Selektion von Chondrozyten nach einem der vorstehenden Patentansprüche, **dadurch gekennzeichnet, dass** im Vergleich zu einem Referenzgen die erfindungsgemäßen Marker bestimmt werden.

8. Verfahren zur Selektion von Chondrozyten nach Anspruch 7, **dadurch gekennzeichnet, dass** mindestens ein Verhältnis der Genexpression ausgewählt aus der Gruppe
| | |
|---|---|
| CRTAC1 / R | > 0,003 |
| NRN1 / R | ≥ 0,1 |
| KAL1 / R | > 1,0 |
| EBF3 / R | ≤ 0,4 |
| ACAN / R | ≥ 0,29 |
bestimmt wird.

9. Verfahren zur Selektion von Chondrozyten nach Anspruch 7, **dadurch gekennzeichnet, dass** mindestens ein Verhältnis der Genexpression ausgewählt aus der Gruppe
| | |
|---|---|
| CRTAC1 / R | ≥ 0,006, insbesondere ≥ 0,009 und ≥ 0,012 |
| NRN1 / R | ≥ 0,2, insbesondere ≥ 0,3 und ≥ 0,4 |
| KAL1 / R | ≥ 2,0, insbesondere ≥ 3,0 und ≥ 4,0 |
| EBF3 / R | ≤ 0,3, insbesondere ≤ 0,2 und ≤ 0,1 |
| ACAN / R | ≥ 0,39, insbesondere ≥ 0,49 und ≥ 0,59 |
bestimmt wird.

10. Verfahren zur Selektion von Chondrozyten nach einem der vorstehenden Patentansprüche, **dadurch gekennzeichnet, dass** der Anteil an Synoviozyten in den isolierten Chondrozyten nach Vermehrung oder den erhaltenen Sphäroiden im Verhältnis zur Gesamtzellzahl weniger oder gleich 9 %, insbesondere weniger oder gleich 5 % beträgt.

11. Isolierte Chondrozyten erhältlich durch ein Verfahren gemäß einem der Ansprüche 1 bis 10.

12. Isolierte Sphäroiden erhältlich durch ein Verfahren gemäß einem der Ansprüche 1 bis 10.

13. Isolierte Sphäroiden erhältlich durch ein Verfahren gemäß einem der Ansprüche 1 bis 10, **dadurch gekennzeichnet, dass** der Anteil an Synoviozyten in den Sphäroiden im Verhältnis zur Gesamtzellzahl weniger oder gleich 9 % beträgt.

14. Transplantierbares Knorpelgewebe enthaltend Chondrozyten und / oder Sphäroiden nach einem der Ansprüche 10 bis 13 oder erhältlich nach einem Verfahren gemäß einem der Ansprüche 1 bis 9.

15. Transplantierbares Knorpelgewebe nach Anspruch 14 zur Verwendung in der Behandlung von Gelenkknorpeldefekten, autologen Chondrozytentransplantation.
